Europäisches Patentamt

European Patent Office

Office européen des brevets

(19)

(11) Veröffentlichungsnummer: **0 386 563 B1**

# (12) EUROPÄISCHE PATENTSCHRIFT

(45) Veröffentlichungstag der Patentschrift: **27.04.94**

(51) Int. Cl.5: **C12N 15/48**, C07H 21/04, A61K 31/70

(21) Anmeldenummer: **90103641.8**

(22) Anmeldetag: **25.02.90**

(54) **Antisense-Oligonukleotide zur Inhibierung der Transaktivatorzielsequenz (TAR) und der Synthese des Transaktivatorproteins (tat) aus HIV-I und deren Verwendung.**

(30) Priorität: **09.03.89 DE 3907562**

(43) Veröffentlichungstag der Anmeldung:
**12.09.90 Patentblatt 90/37**

(45) Bekanntmachung des Hinweises auf die Patenterteilung:
**27.04.94 Patentblatt 94/17**

(84) Benannte Vertragsstaaten:
**AT BE CH DE ES FR GB GR IT LI LU NL SE**

(56) Entgegenhaltungen:
**WO-A-88/07544**

**PROCEEDINGS OF THE NATIONAL ACADEMY OF SCIENCES OF THE UNITED STATES OF AMERICA, Band 85, Nr. 20, Oktober 1988, Baltimore, USA; P.S. SARIN et al., Seiten 7448-7451**

**JOURNAL OF VIROLOGY, Band 62, Nr. 10, Oktober 1988, Washington, DC; J.A. ZAIA et al., Seiten 3914-3917**

(73) Patentinhaber: **BAYER AG**

**D-51368 Leverkusen(DE)**

(72) Erfinder: **Stropp, Udo, Dr.**
**Breidenhofer Strasse 12**
**D-5657 Haan(DE)**
Erfinder: **Baumgarten, Jörg, Dr.**
**Henselweg 13**
**D-5600 Wuppertal 1(DE)**
Erfinder: **Löbberding, Antonius, Dr.**
**Am Rohm 105**
**D-5600 Wuppertal 1(DE)**
Erfinder: **Springer, Wolfgang, Dr.**
**Katernberger Schulweg 31**
**D-5600 Wuppertal 1(DE)**
Erfinder: **Piel, Norbert, Dr.**
**Millrather Weg 74**
**D-4006 Erkrath(DE)**
Erfinder: **Kretschmer, Axel, Dr.**
**Richard-Zörner-Strasse 32**
**D-5060 Bergisch-Gladbach 1(DE)**

EP 0 386 563 B1

2

ANALYTICAL BIOCHEMISTRY, Band 172,
1988, San Diego, USA; C. J. MARCUS-SEKU-
RA, Seiten 289-295

CANCER RESEARCH, Band 48, Nr. 10, 1988,
Chicago; A. STEIN et al., Seiten 2659-2668

Erfinder: **Kölbl, Heinz, Dr., z.Zt. Mobay Corporation
Animal Health Division,
9009 West 67th Street
Merriam, Kansas 66202/3632(US)**
Erfinder: **Frommer, Werner, Prof. Dr.
Claudiusweg 17
D-5600 Wuppertal 1(DE)**

**Beschreibung**

Das gezielte Abschalten ungewollter Genexpression durch komplementäre Nukleinsäuren ist ein sehr wirksames Instrument, genetisch bedingte Störungen einer Proteinüberproduktion zu beheben. Hierzu gehört in einem weiteren Sinne auch das Abschalten fremder Nukleinsäuren in einem Organismus, wie nach einem Befall mit Viren, Bakterien oder Pilzen. Bei der Antisense-Methodik hybridisieren Antisense-Oligonukleotidderivate mit der zu hemmenden mRNA, so daß eine Proteinsynthese ausgehend von dieser mRNA unterbunden wird. Der Mechanismus der Hemmwirkung ist dabei noch nicht endgültig aufgeklärt. Diskutiert wird ein Abbau der RNA an den hybridisierten Stellen mit RNAse H oder eine sterische Hinderung der Ribosomenbindung, sowie eine Inhibierung des Spleißvorgangs. In der Literatur gibt es Belege für die Wirkung von Antisense-Oligonukleotiden (Markus-Sekura, C.J. (1988) Analytical Biochem. 172, 289-295; Stein, C.A. und Cohen, J.S. (1988) Cancer Research 48, 2659-2668; Paoletti. C. (1988) Anti-Cancer Drug Design 2, 325-331; Loose-Mitchell, D.S. (1988) Trends in Pharmacological Sciences 9, 45-47).

In der hier vorliegenden Erfindung wurde das HIV-I-Tat-Gen als Ziel der Antisense-Strategie ausgewählt, weil das Tat-Protein ein essentielles Regulatorprotein der Virusvermehrung ist (Dayton, A.J. et.al. (1986) Cell 44, 941-947, Fisher, A.G. et.al. (1986) Nature 320, 367-369). Desweiteren wurde die HIV-I-Transaktivatorzielregion (TAR) im LTR (long terminal repeat) ausgewählt, weil sie allen bisher bekannten mRNAs des Virus gemeinsam ist (Rosen, C.A. et.al. (1985 Cell 41, 813-823, Parkin, N.T. et.al. (1988) EMBO J. 7, 2831-2837).

Die erfindungsgemäßen Antisense-Oligonukleotide sind chemisch modifizierte Oligonukleotide und Oligonukleoside, die gegenüber normalen Desoxyribonukleinsäureoligomeren insbesondere an den Phosphordiesterbindungen und auch an den 3'- und 5' Termini derivatisiert sind, so daß sie resistenter gegen Nuclease-Abbau sind und bessere Permeationseigenschaften oder Hybridisierungseigenschaften aufweisen. Diese chemisch modifizierten Antisense-Oligonukleotide werden im folgenden Text kurz als Antisense-Oligonukleotide bezeichnet. Diese erfindungsgemäßen Antisense-Oligonukleotide sind solche, die eine Hemmung der Synthese des Transaktivatorproteins und der Transaktivatorzielsequenz (TAR) aus HIV-I (AIDS-Virus) bewirken und damit antivirale Aktivität besitzen.

In jüngster Zeit wurden einige Forschungsarbeiten zum Versuch des Nachweises der Inhibierung der Expression von HIV-I Genen durch Antisense-Oligonukleotide veröffentlicht [s. Agrawal et al., Proc. Natl. Acad. Sci., USA 85, 7079-7083 (1988); J. Goodchild et al., Proc. Natl. Acad. Sci., USA, 85, 5507-5511 (1988); J. Zaia et al., J. Virol. 62, 3914-3917 (1988); P. Sarin et al., Proc. Natl. Acad. Sci., USA 85, 7448-7451 (1988); M. Matsukura et al., Proc. Natl. Acad. Sci., USA 84, 7706-7710 (1987)].

Wir haben die in den Publikationen aufgeführten Antisense-Oligonukleotide ebenfalls in unseren Tests geprüft und fanden heraus, daß die biologische Aktivität der Antisense-Oligonukleotide mit den von uns beanspruchten Nukleinsäuresequenzen eine deutlich höhere inhibitorische Aktivität aufwiesen.

Außerdem zeigte sich, daß normale Desoxyribonukleinsäureoligonukleotide in Zellkulturtests in 2-3 Stunden fast vollständig abgebaut werden und daher die von uns eingesetzten, chemisch modifizierten Antisense-Oligonukleotide durch ihre hohe Nukleaseresistenz den publizierten Wirkungen [(J. Goodchild et al., Proc. Natl. Acad. Sci., USA 85, 5507-5511 (1988)] überlegen sind.

Versuche, die zur Auffindung antiviraler Oligonukleotide gegen HIV führten:
In einem gekoppelten in vitro Test wird ausgehend von dem Transaktivator-Genfragment aus HIV-I Tat-mRNA gebildet. Anschließend wird von der mRNA in einem Kaninchen-Retikulozytenlysat Protein synthetisiert. Dieser Schritt der Proteinsynthese wird durch Antisense-Oligonukleotide kontrolliert. Sie hybridisieren dabei sequenzspezifisch mit der Tat-mRNA und verhindern hierdurch die Proteinsynthese. Über den exakten Mechanismus der Hemmwirkung ist noch nichts Genaues bekannt.

Im Falle von Antisense-mRNA wird die sense mRNA vollständig durch RNA/RNA-Duplexbildung blockiert und ist für eine Proteinsynthese nicht mehr zugänglich. Bei Verwendung von geeigneten Antisense-Oligonukleotiden gibt es eine Vielzahl von theoretischen Möglichkeiten, eine vorhandene mRNA zu blockieren. Obwohl der exakte Hemmechanismus nicht bekannt ist, kann man auf der Ziel-mRNA "sensible" Regionen postulieren, in denen Antisense-Oligonukleotide ihre gewünschte Wirkung zeigen können. Diese sensiblen Regionen können unter anderem die Ribosomenbindungsstelle und der Bereich um das AUG-Startcodon sein. Es können aber auch Regionen sein, die durch die Sekundärstruktur der mRNA bedingt sind, wie z.B. Stamm- und Loop-Bereiche. Sensibel können auch Bereiche auf der mRNA sein, die mit Proteinen oder anderen Nukleinsäuren wechselwirken. Hierzu gehören auch die Spleißstellen.

Die Wirkung der Antisense-Oligonukleotide wurde mit dem folgenden Test überprüft:
1. In vitro Transkription:
ausgehend vom zu hemmenden Gen wird mRNA gebildet

2. In vitro Translation:

von der in vitro synthetisierten mRNA wird Protein synthetisiert

3. Hemmung der in vitro Translation:

nachdem unter 2. die Proteinsynthese erfolgt ist (positive Kontrolle), können Antisense-Oligonukleotide eingesetzt werden, um die Proteinsynthese in nun folgenden Tests zu hemmen und die Hemmung quantitativ zu messen.

4. Hemmung in der Zellkulltur:

die unter 3. positiv aufgefallenen Antisense-Oligonukleotide werden in der Zellkultur getestet.

Es wird ferner darauf geachtet, daß die Antisense-Sequenzen in hoch konservierten Gensequenz-Regionen liegen. Nach derzeitigem Kenntnisstand sind die erfindungsgemäßen Oligonukleotide und deren Derivate, wie unten beschrieben für die parenterale Anwendung geeignet. Die entsprechenden pharmazeutischen Zubereitungen enthalten neben den Oligonukleotiden die für parenterale Zubereitungen üblichen Hilfsstoffe wie mm Beispiel Puffer und/oder Stabilisatoren oder Liposomenformulierungen. Ferner ist eine topische Anwendung denkbar, weil das tat-Gen in der Haut exprimiert wird und hier möglicherweise Karposi-Sarkome verursacht [J. Vogel et al., Nature 335, 606-611 (1988)]. Die hierfür einsetzbaren Zubereitungen sind mm Beispiel Salben, Cremes, Lösungen oder Pflaster, die neben dem Wirkstoff die für diese Anwendung geeigneten pharmazeutischen Hilfsstoffe enthalten.

Beispiel 1:

Synthese chemisch modifizierter Antisense-Oligonukleotide

Synthese von modifizierten und unmodifizierten Antisense-Oligonukleotiden:

I. Darstellung von Olgonukleotiddiestern mittels automatischer Gensynthese nach der Phophoramidit-Methode: Beaucage et al., Tetrahedron Lett. 22, 1859 (1981)

II. Darstellung der Phosphorthioate mittels automatischer Oligonukleotidsynthese ausgehend von Hydrogenphosphonat-Vorstufen oder Phosphoramidaten:

Stec et al., J. Am. Chem. Soc. 106, 6077 (1984)

B. Froehler, Tetrahedron Lett. 27, 5575-5578 (1986)

III. Darstellung der Methylphosphonate:

P. Miller et al., Biochemistry 25, 5092-5097 (1986)

IV. Darstellung von Oligonukleosiden mit Carbamatinternukleosidbindung

E. Stirchak et al., J. Org. Chem. 52, 4202-4206 (1987)

V. Darstellung von Oligonukleotiden, wobei diese an ausgewählten Internukleosidbindungen

    a) eine oder mehrere Phosphorthioatbindungen gemäß II

    b) eine oder mehrere Methylphosphonatbindungen gemäß III

    c) eine oder mehrere Carbamatinternukleosidbindungen gemäß IV

    d) mehrere Internunkleosidbindungen gemäß I, II, III und IV gleichzeitig

aufweisen.

VI. Darstellung von Oligonukleotiden, die kovalent am 3'- oder 5' Terminus mit Verbindungen verbunden sind:

M. Lemaitre et al., Nucleosides Nucleotides 6, 311-315 (1987)

N.T. Thuong et al., PNAS (USA) 84, 5129-5133 (1987)

T. Le Doan et al., Nucleic Acid Res. 15, 7749-7760 (1987)

J. Vasseur et al., Biochem. Biophys. Res. Comm., 152, 56-61 (1988)

E. Brosalind et al., Bioorg. Khim. 14, 125-128 (1988)

J.J. Toulme et al., Proc. Natl. Acad. Sci., USA, 83, 1227 (1986)

Beispiel 2

Klonierung von Genfragmenten in den Vektor pSP65:

pSP65-Tat: das 240 bp große Sal I/ Hind III-Tat-Fragment aus HIV-I (nt 5358 - 5625, nach L. Ratner et al. Nature, 313 (1985), 277-284) wurde in den identisch geschnittenen pSP65 Vektor ligiert und in E.coli 5k transformiert (Maniatis, T. et al. (1982) "Molecular Cloning", Cold Spring Harbor Laboratory, Cold Spring Harbor, New York)

pSP65-TAR-tat: das TAR-Fragment [(-17, 80), bezogen auf die Cap-Stelle] wurde aus der LTR des HIV-I durch Schneiden mit Pvu II und Sal I isoliert und in die Sma I/ Sal I- Schnittstelle des Konstruktes pSP65-Tat ligiert. (Das Tat-Gen dient in dem pSP65-TAR-Tat-Klon nur als Reportergen und könnte auch durch ein anderes Protein ersetzt werden.)

Positive Klone wurden durch Plasmidminipräparation und Restriktionsenzymverdau identifiziert und anschließend durch DNA-Sequenzanalyse vollständig charakterisiert.

Beispiel 3:

Gekoppelte in vitro Transkription und Translation

Tat-mRNA bzw. TAR-mRNA wurden im wesentlichen nach Melton, D.A. et.al. (1984) Nucl. Acids Res. 12, 7035-7056 synthetisiert, wobei die DNA der Konstrukte mit Pvu II oder Hind III linearisiert wurden.

Zur in vitro Translation wurde ein Kaninchenretikulozytenlysat der Fa. Stratagene, La Jolla, CA, USA verwendet, das im wesentlichen auf den Methoden von Hunt, T. und Jackson, R.J. (1974) "Modern Trends in Human Leukemia", Hrsg. Neth, Gallo, Spiegelman und Stohlman, Lehmannns Verlag, München und Pelham, H.R.B. und Jackson, R.J. (1976) Eur. J. Biochem. 67, 247ff beruht.

1 μg mRNA wird in Gegenwart von radioaktiv-markiertem Cystein zur Translation eingesetzt. Die Analyse des gebildeten Proteins erfolgte auf einem 6-20 % diskontinuierlichem SDS-PAGE nach Laemmli, U.K. (1970) Nature 227, 680-685.

Zur quantitativen Messung der Translationsinhibition durch Antisense-Oligonukleotide wurden zur mRNA eine gewünschte Menge Oligonukleotid (0,1 μg - 2 μg) hinzugegeben und dann wie oben beschrieben translatiert. Autoradiographien von SDS-Polyacrylamidelektrophoresegelen der Testansätze wurden mit einem Scanner quantitativ ausgewertet. Wie aus der Tabelle 1 hervorgeht, hemmen die eingesetzten spezifischen Antisense-Oligonukleotide die Translation zu 90-95%. Unspezifische Antisense-Oligonukleotide hemmen dagegen nicht.

Beispiel 4:

Klonierung eines LTR-hGH-Reportergenkonstruktes zur Transfektion in eukaryotischen Zellen

Aus dem Plasmid pOGH (Nichols Institute, Los Angeles, CA, USA) wurde das genomische hGH-Genfragment (humanes Wachstumshormongen) mit seinen 5 Exons und Introns als 2.2kb großes Sal I-EcoRI Fragment herausgeschnitten.

Gleichzeitig wurde aus dem Plasmid pΔ(83-5365/5607-7719/8053-9296) ein 2.1kb großes EcoRI-Sal I Fragment herausgeschnitten, das das LTR-Fragment von HIV(-167,80) und pBR322-Sequenzen mit dem Replicon enthält (Sodroski, J. et al., Science (1985) 229, 74-77).

Durch die Doppelspaltung mit EcoRI und Sal I erhält man die richtige Orientierung des hGH-Gens zum LTR-Promoterbereich. Beide Genfragmente wurden miteinander ligiert und in E.coli 5K transformiert.

Rekombinante Klone, die LTR und hGH-Sequenzen enthalten, wurden über die Ampicillinresistenz von pBR322 vorselektioniert und dann nach analytischer Isolierung der Plasmide durch Spaltung mit Restriktionserzymen und Southern Blotting mit dem LTR- und hGH-Genfragment als Genprobe sowie durch DNA-Sequenzanalyse identifiziert.

Beispiel 5:

Herstellung einer Testzellinie

Die biologische Wirkung von antisense Oligonukleotiden wurde mit einer genetisch manipulierten HeLa-Zellinie gemessen. Hierzu wurde der LTR-hGH enthaltende Vektor zusammen mit pKOneo stabil in die HeLa-Zellinie (ATCC.CCL2) cotransfiziert.

Die Cotransfektion wurde nach der Ca-Phosphat Methode durchgeführt, wie in "Basic Methods in Molecular Biology", Elsevier Science Publishing Co, Inc. New York,(1986), Seiten 286-289 beschrieben.

Die Selektion der cotransfizierten Zellen erfolgte mit dem Antibiotikum G418 Sulfat (Fa. Gibco). Nach der Cotransfektion mit dem LTR-hGH-Vektor und dem pKOneo-Vektor, das das G418 Resistenz-Gen enthält und einer 2-tägigen Vorinkubation wurde dem Wachstumsmedium (DMEM, Fa Flow Lab.) 0,6 mg G418 pro ml zugesetzt. Nach weiterer 14-tägiger Inkubation wurden die Klone isoliert, die sich aus den gegen G418 resistenten transfizierten Zellen gebildet hatten.

Die isolierten Klone wurden auf hGH-Produktion untersucht. Der stabile Einbau der LTR-Region wurde mit Hilfe von Genprobetechniken detektiert, d.h. die genomische DNA der Zellen wurde isoliert und die LTR-Region mithilfe der PCR-Methode (polymerase chain reaction) amplifiziert und mit LTR-spezifischen Gensonden nachgewiesen. Zwei HeLa-Transfektanten wurden aufgrund dieser Untersuchungen als Testzellinien ausgewählt.

Beispiel 6:

Biologische Testung von Oligonukleotiden

Die biologische Wirkung von Antisense-Oligonukleotiden, die spezifisch auf die LTR-Region aus HIV einwirken, wurde an der Hemmung der Bildung des humanen Wachstumshormons (hGH) durch die Testzellinien gemessen. Die hGH-Testung erfolgte mit dem von der Fa. Nichols Institute, Diagnostics, San Juan Capistrano angebotenen Immunoassay zur quantitativen Bestimmung von humanem Wachstumshormon.

Die HeLa-Testzellimen wurden zunächst für 1 bis 3 Tage in Gegenwart verschiedener Konzentrationen (10 $\mu$g - 100 $\mu$g/ml) an antisense Phosphorthioat-Oligonukleotiden vorinkubiert. Anschließend wurden die Zellen in Gegenwart der Oligonukleotide trypsiniert und in neues Wachstumsmedium (DMEM Medium, Fa. Flow Lab.) umgesetzt. Nach weiterer Inkubation für 24 und 48 Stunden wurde die hGH-Konzentration im Wachstumsmedium gemessen. Der Test wurde in 96 microwell Mikrotiterplatten durchgeführt.

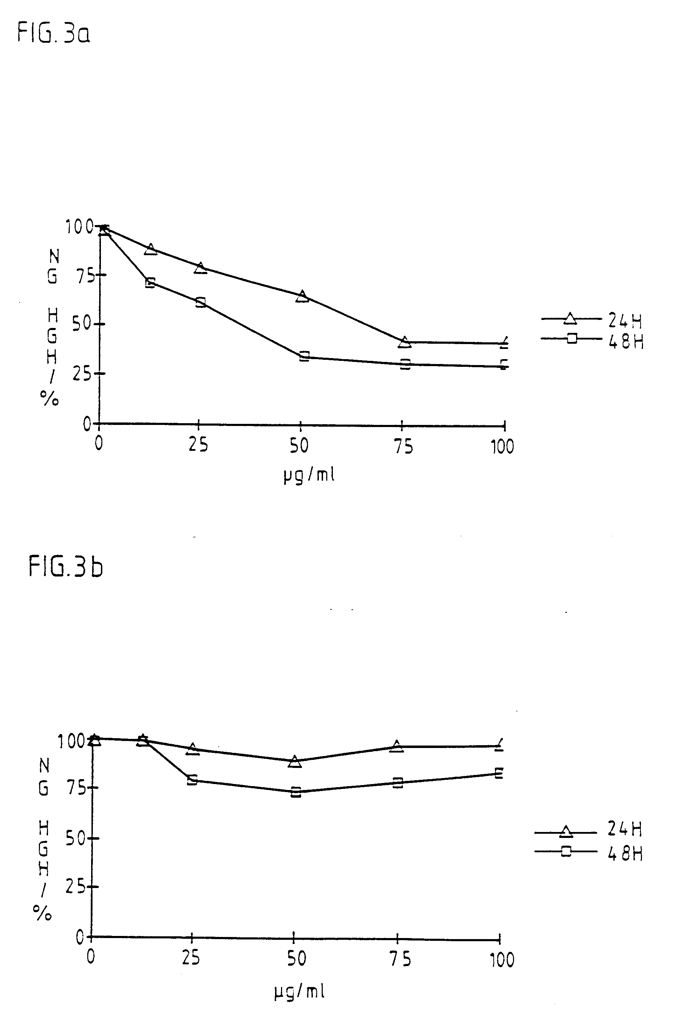

Wie aus der Abb. 3a zu entnehmen ist, hemmen die LTR sequenzspezifischen antisense Oligonukleotide (g) und (h) konzentrationsabhängig die hGH Produktion zwischen 30 und 70%.

Ein nicht sequenzspezifisches Phosphorthioat-Oligonukleotid (CCC GCG CCA AAG ACC GCC ACC), bei dem alle Phosphordiesterbindungen schwefelsubstituiert sind, zeigte dagegen auch in höheren Konzentrationen (50-100 $\mu$g/ml) nur geringe inhibitorische Aktivität (Abb. 3b).

Ergebnisse

Die in vitro Translation der Tat-mRNA führte zu einem Protein von der Größe 13 kDa (bei Hind III geschnittener DNA) und 17 kDa (bei Pvu II geschnittener DNA).

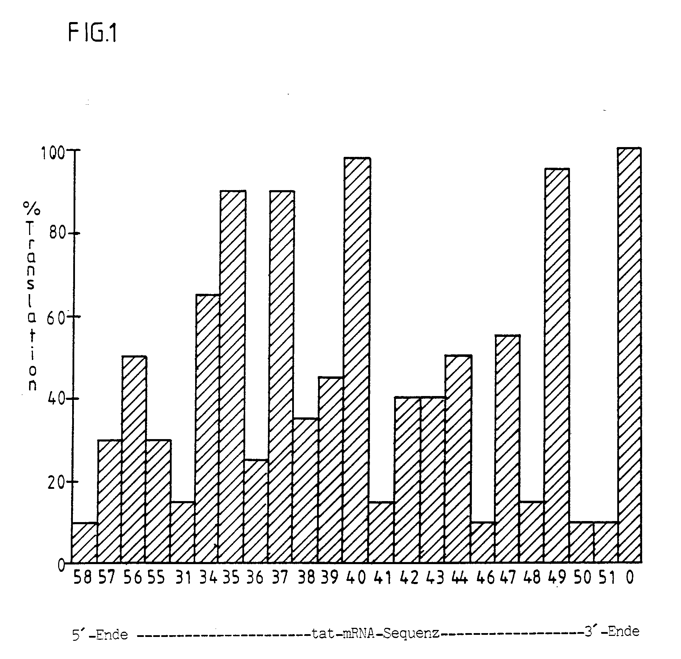

Die Hemmung der in vitro Translation durch Antisense-Oligonulcleotid-Diester erfolgte bei einer Konzentration von ca 5-25 $\mu$M Oligonukleotid. Es wurden Oligonukleotide ausfindig gemacht, die eine Hemmung der Proteinsynthese von ca. 90-95 % erzielten (siehe Tabelle 1 und Abb. 1).

Das Resultat der Hemmung der in vitro Translationen ist in der Abbildung 1 dargestellt. Gebildetes Protein in Prozent ist gegen die jeweiligen Antisense-Oligonukleotide aufgetragen. Die sequenzabhängige Lage der Antisense-Oligonukleotide ist schematisch auf der Abzisse in 5'3'-Orientierung der Tat-mRNA aufgeführt. Die Kontrolle (in der Abb.1 ganz rechts dargestellt) symbolisiert 100 % Tat-Proteinsynthese. Ein Oligonukleotid wirkt um so besser, je geringer die Proteinsynthese ist.

Es wurden Antisense-Oligonukleotide gegen die folgenden TAR- und Leadersequenzen nt 21-53, 74-161, 202-279 und das zweite und dritte Exon des Tat-Gens nt 5368-5403, 5421-5548, 5583-5617 und nt 7967-8366, 8385-9183 (Nukleotid-Nummern nach L. Ratner et al., Nature 313, (1985) 277-284) des HIV I Genoms gefunden.

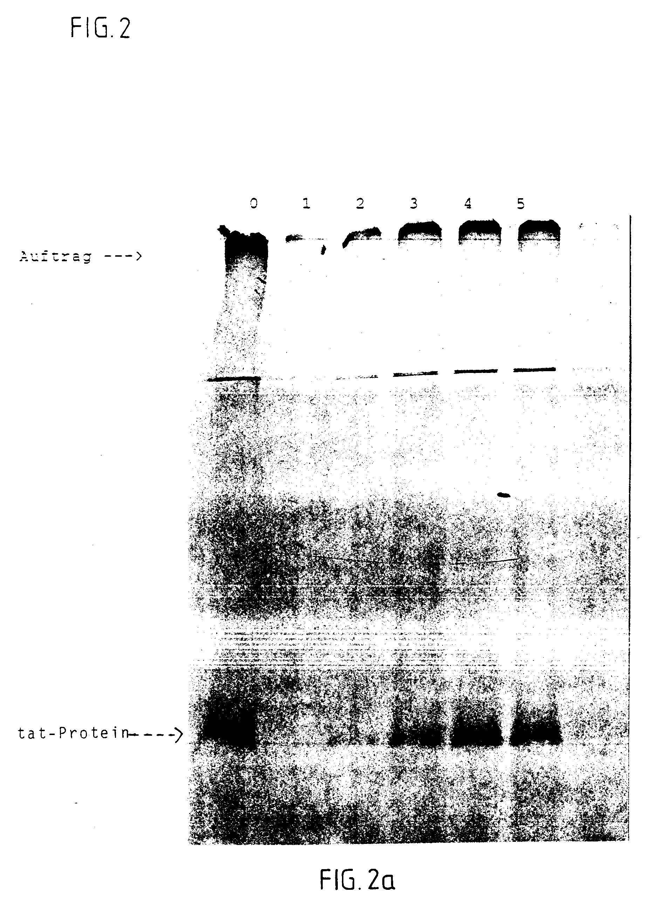

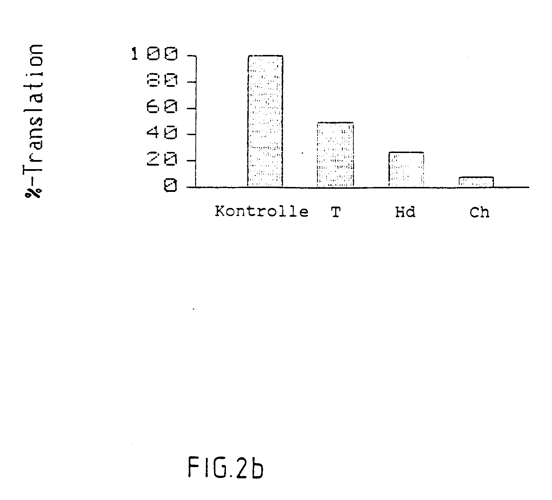

Bei der durchgeführten Testreihe war es überraschend, daß die Antisense-Oligonukleotide sowohl in der 5' nicht translatierten Region, am 3'-Ende der vorliegenden Tat-mRNA und innerhalb der Tat-mRNA die Proteinsynthese hemmen können, obwohl andere Gruppen publiziert haben, daß die Antiserse-Oligonukleotide im Bereich des AUG-Startcodons die Proteinsynthese am besten hemmen. Die hier vorgestellten Experimente zeigen überraschenderweise, daß Oligonukleotide auch am Ende der mRNA eine sehr gute Inhibierung zeigen Der Mechanismus, der diesem Phänomen zugrunde liegt, ist unbekannt. Die Antisense-Oligonukleotide zeigen eine deutliche Dosis-Wirkungs-Beziehung auf die tat-Expression in vitro (Abb. 2). Experimentell konnte bei gleicher Vorgehensweise ebenso die inhibitorische Wirkung von chemisch modifizierten Anisense-Oligonukleotiden nachgewiesen werden: Die entsprechenden Methylphosphonate und Phosphorthioate korrelierten sehr gut in ihrer Wirkung mit den genannten Ergebnissen, die mit Phosphordiester-Oligonukleotiden erzielt wurden.

Als besonders hoch wirksam erweist sich eine Kombination aus internukleotid-modifizierten Oligonukleotiden mit endständig kovalent verknüpften lipophilen Verbindungen wie Cholesterin, Hexadekanol, Acridinderivate u.a (Synthese analog zu Thuong, N.T. und Chassignol, M. (1988), Tetrahedron Letters 29, 5905-5908). Diese Konjugate erhöhen die Zellmembranpenetration unter gleichzeitiger Wirkungssteigerung. So Zeigen beispielsweise Konjugate aus Phosphorthioat-Oligonukleotiden und Cholesterin eine deutlich höhere

6

Hemmung der Proteinsynthese als das identische Phosphorthioat ohne das kovalent gebundene Cholesterin (Abb. 2A).

Die Wirkung der HIV-sequenzspezifischen Antisense-Oligonukleotide wurde auch an Östrogenrezeptor-, TMV- und BMV-mRNA erprobt. Es zeigte sich, daß sie die Translation dieser mRNAs in den verwendeten Konzentrationen nicht beeinflussen. Die erfindungsgemäßen Oligonukleotide sind also hoch spezifisch für die HIV-Gensequenz.

Die hier ebenfalls getestete TAR-mRNA zeichnet sich durch eine ausgeprägte Stamm-Loop-Struktur aus (Feng, S. und Holland, E.C. (1988) Nature 334, 165-167). In dieser Transaktivatorzielsequenz TAR wirkten die Oligonukleotide (Phosphorthioate) am besten, die im Loop-Bereich hybridisierten (g und h in Tabelle 1). Die Sekundärstruktur kann aus einer computer-simulierten Berechnung erhalten werden.

Vergleichende Untersuchungen hinsichtlich der Anzahl der Nukleotidmonomeren in den Antisense-Oligonukleotiden haben ergeben, daß insbesordere ab einer Sequenzlänge von 10 Nukleotiden sehr gute Wirkungen zur Translationsinhibietung erzielbar waren. Weiterhin wurde festgestellt, daß bei Antisense-Oligonukleotiden mit einer Sequenzlänge von mehr als 18 Nukleotiden keine Steigerung der Wirkung in den von uns verwendeten Tests nachweisbar war, aber auch keine Verringerung der Translationsinhibierung auftrat. Die Verwendung von längerkettigen Antisense-Oligonukleotiden ist für die sequenzspezifische Wirkung und die Hybridisierung mit einer möglichst hohen Schmelztemperatur (Tm) förderlich. Daher können grundsätzlich die von uns als besonders wirksam identifizierten Antisense-Oligonukleotide an den angegeben 3'- und 5'-Termini der Target-Sequenz entsprechend durch die komplementären Nukleotide verlängert werden, ohne die gefundene Wirkqualität zu verlieren. Die erfindungsgemäße Wirkqualität kann außerdem durch Veränderungen in den angegebenen Nukleotidsequenzen durch Austausch einer oder mehrere Nukleobasen mit anderen Partialstrukturen wie z.B. Hypoxanthin oder anderen Purinen, Pyrimidinen oder gänzlich anderen Substituenten aufrecht erhalten werden, die die Hybridisierung nicht verschlechtern. Weiterhin wirken Antisense-Oligonukleotide bei Veränderungen der angegebenen Oligonukleotid-Sequenzen durch Weglassen oder Hinzunahme von Nukleotiden oder Pseudonukleotiden innerhalb der genannten Sequenzen, wenn die Hybridisierung dadurch nicht verhindert wird.

An das Zellfreie in vitro Testsystem schließt sich ein Zelltestsystem an, in dem die "ex vivo" Wirkung der Antisense-Oligonukleotide bestätigt wird. Hierzu werden aber keine normalen Diester-Oligonukleotide verwendet, weil sie schlecht die Zellmembran penetrieren können und im Zytosol zu schnell durch Nukleasen abgebaut werden würden. An die Stelle der Diester werden im Zelltest Methylphosphonate, Phosphorthioate oder andersartig, gegen Nukleasen geschützte Oligonukleotide verwendet, die ferner die Fähigkeit besitzen müssen, Zellmembranen penetrieren zu können. Zahlreiche Modifizierungen wurden beschrieben:

Methylphosphonate: Miller, P.S. et. al. (1985) Biochimie 67, 769-776; Phosphorthioate: F. Eckstein, Ann. Rev. Biochem. 54, 367-402, Matsukura, M. et. al. (1987) PNAS 84, 7706-7710; endständig modifizierte Oligonukleotide (polyL-Lys, Acridine, Psoralene, Azidoproflavin): Lemaitre, M. et.al. (1987) PNAS 84, 648-652, Zerial, A. et.al. (1987) Nucleic Acids Res. 15, 9909-9919; Toulme, J.J. et al. (1986) PNAS 83, 1227-12231; Lee, B.L. et al. (1988) Biochemistry 27, 3187-3203; Le Doan, T. et al. (1987) NAR 15, 7749ff.; Alpha-Oligonukleotide: Thuong, N.T. et.al. (1987) PNAS 84, 5129-5133; Carbamat-Oligonukleotide: Stirchak, E.P. and Summerton, J.E. (1987), J. Organic Chemistry 52, 4202-4206, Amidat-Oligonukleotide: diese und andere Derivate werden in den Übersichtsartikeln von G. Zon aufgeführt (Pharmaceutical Research 5(1988), 539-549).

Die folgenden Antisense-Oligonukleotide erwiesen sich aus der Gruppe einer Vielzahl von geksteten Oligonukleotiden als besonders hoch wirksam gegen HIV-I.

Tat-Oligos:

| | | | |
|---|---|---|---|
| a) | TAA CGC CTA TTC TGC TAT GTC GAC | (5366-5389) |
| b) | TCT AGT CTA GGA TCT ACT GG | (5417-5436) |
| c) | GGC TGA CTT CCT GGA TGC TT | (5444-5463) |
| d) | GCA ATG AAA GCA ACA CTT TT | (5493-5512) |
| e) | GAG TCT GAC TGC CTT GAG GA | (5584-5603) |
| f) | CTT TGA TAG AGA AAC TTG AT | (5604-5623) |

TAR-Oligos:

| | | | |
|---|---|---|---|
| g) | GCT CCC AGG CTC AGA TC | (21-37) |
| h) | CCA GAG AGC TCC CAG GC | (28-44) |

Die Numerierung erfolgt nach L. Ratner (siehe oben).

Die vorliegende Erfindung umfaßt auch Fragmente dieser Nukleotide, sowie Verlängerungen dieser Sequenzen am 3'- und/oder 5'-Ende, insofern sie die Wirkung der angegebenen Sequenzen nicht verschlechtern.

Außerdem Veränderungen in den angegebenen Nukleotidsequenzen durch Austausch einer oder mehrerer Nukleobasen mit anderen Partialstrurturen wie z.B. Inosin oder anderen Purinen, Pyrimidinen oder gänzlich anderen Substituenten.

Außerdem Veränderungen der angegebenen Oligonukleotidsequenzen durch Weglassen oder Hinzunahme von Nukleotiden oder Pseudonukleotiden innerhalb der genannten Sequenzen. Im allgemeinen beträgt die Kettenlänge der Oligonukleotide 6-50 Basenpaare. Bevorzugt sind 10-30 und besonders bevorzugt sind 10-20 Basenpaare.

Es ist bekannt, daß das tat-Gen mit dem rev-Gen überlappt. Unabhängig vom Namen des Gens gelten die hier angegebenen Sequenzen als Wirkort für die erfindungsgemäßen Antisense-Oligonukleotide und Derivate.

Die in diesem Patent aufgeführten Sequenzen, einschließlich ihrer Komplemente werden als Gensonden zur Diagnostik von HIV-I in Patienten und Gewebeproben sowie in Blutkonserven eingesetzt. Verwendet werden hierzu Diester oder chemisch modifizierte Oligonukleotide in der Weise, daß das Oligonukleotid mit detektierbaren Substanzen gekoppelt wird (radioaktive Isotope, Enzyme, chemoluminierende oder fluoreszierende Farbstoffe, Biotin oder andere durch Antikörper erkennbare Epitope u.a.).

8

Tabelle 1

| Tabelle der getesteten Oligonukleotide in der Tat-mRNA-Region: | | |
|---|---|---|
| Oligonukleotid (interne Numerierung) | %-Translation | Oligonukleotide mit guter Hemmwirkung und unbeschrieben |
| 58 | 10 | a |
| 57 | 30 | |
| 56 | 50 | |
| 55 | 30 | |
| 31 | 15 | b |
| 34 | 65 | |
| 35 | 90 | |
| 36 | 25 | c |
| 37 | 90 | |
| 38 | 35 | |
| 39 | 45 | |
| 40 | 98 | |
| 41 | 15 | d |
| 42 | 40 | |
| 43 | 40 | |
| 44 | 50 | |
| 46 | 10 | |
| 47 | 55 | |
| 48 | 15 | |
| 49 | 95 | |
| 50 | 10 | e |
| 51 | 10 | f |
| Kontrolle (ohne Oligo) | 100 | |
| Phosphorthioatoligonukleotide in der TAR-Region: | | |
| Oligonukleotid (interne Numerierung) | %-Translation | Oligonukleotide mit guter Hemmwirkung |
| 560 | 20 | |
| 558 | 5 | g |
| 559 | 5 | h |
| 561 | 20 | |
| 562 | 55 | |

**Legende zu Fig. 1**:

Hemmung der tat-Proteinsynthese durch Antisense-Oligonukleotide:

gebildetes Protein in Prozent ist gegen die jeweiligen Antisense-Oligonukleotide mit einer internen Numerierung aufgetragen. Die sequenzabhängige Lage der Antisense-Oligonukleotide ist schematisch auf der Abzisse in 5'-3'-Orientierung der tat-mRNA aufgeführt. Die Kontrolle (in der Abb. 1 ganz rechts dargestellt) symbolisiert 100 % tat-Proteinsynthese. Ein Antisense-Oligonukleotid wirkt um so besser, je geringer die Proteinsynthese (Translation) ist.

**Legende zu Fig. 2a**

Dosis-Wirkungs-Beziehung eines Oligonukleotids auf die tat-Expression in vitro:

0 Kontrolle ohne Oligonukleotid

1 2000 ng Oligonukleotid a

2 1000 ng Oligonukleotid a

3 500 ng Oligonukleotid a

4 100 ng Oligonukleotid a

5 10 ng Oligonukleotid a

**Legende zu Fig. 2b**

Wirkungsvergleich eines Phosphorthioat-Oligonukleotids (a) mit Sequenzgleichen 5'-Konjugaten aus Cholesterin oder Hexadekanol

Wirkungsvergleich eines Phosphorthioat-Oligonukleotids (T) mit sequenzgleichen 5'-Konjugaten aus Cholesterin (Ch) oder Hexadekanol (Hd). 1 μg tat-RNA wurde in Gegenwart von 0,8 μg Antisense-Oligonukleotid mit einem Retikulozytenlysat translatiert (siehe Methode: Beispiel 3), die Proteinsynthese ohne zugesetztes Oligonukleolid diente dabei als Bezugswert (Kontrolle der Protheinsynthese).

Fig. 3a

Hemmung der hGH-Produktion in der mit dem Plasmid LTR-hGH transfizierten Testzellinie durch sequenzspezifische Phosphorthioate (g oder h) in Abhängigkeit der Konzentration

Fig 3b

Kontrolle: Wirkung einer unspezifischen Sequenz auf die hGH-Produktion

**Patentansprüche**

1. Chemisch modifizierte Antisense-Oligonukleotide mit Sequenzen gegen die TAR-Sequenz und Leader-Sequenz nt 21-53, 74-161, 202-279 und das zweite und dritte Exon des Tat-Gens nt 5368-5403, 5421-5548, 5583-5617 und nt 7967-8366, 8385-9183 des HIV-I-Genoms.

2. Antisense-Oligonukleotide gemäß Anspruch 1 gegen die TAR-Sequenz nt 21-53 und Tat-Sequenz 5368-5403, 5421-5548, 5583-5617.

3. Antisense-Oligonukleotide gemäß den Ansprüchen 1 und 2 aus der Gruppe:

| | | |
|---|---|---|
| a) | TAA CGC CTA TTC TGC TAT GTC GAC | (5366-5389) |
| b) | TCT AGT CTA GGA TCT ACT GG | (5417-5436) |
| c) | GGC TGA CTT CCT GGA TGC TT | (5444-5463) |
| d) | GCA ATG AAA GCA ACA CTT TT | (5493-5512) |
| e) | GAG TCT GAC TGC CTT GAG GA | (5584-5603) |
| f) | CTT TGA TAG AGA AAC TTG AT | (5604-5623) |
| g) | GCT CCC AGG CTC AGA TC | (21-37) |
| h) | CCA GAG AGC TCC CAG GC | (28-44) |

4. Antisense-Oligonukleotide gemäß den Ansprüchen 1 bis 3, worin eine, mehrere oder alle Intermukleotidphosphordiesterbindungen als Phosphorthioate oder Methylphosphonate oder beide chemischen Modifikationen vorliegen.

5. Arzneimittel enthaltend die Antisense-Oligonukleotide gemäß den Ansprüchen 1 bis 4.

6. Antisense-Oligonukleotide gem. den Ansprüchen 1 bis 4 zur Anwendung zur therapeutischen Behandlung von HIV-Infektionen.

7. Verwendung der Antisense-Oligonukleotide nach Ansprüchen 1 bis 4 einschließlich der komplementären Sequenzen als Gensonde.

8. Verwendung von Antisense-Oligonukleotiden mit Sequenzen gemäß Anspruch 3 einschließlich deren komplementären Sequenzen als Gensonden, wobei die als Diester vorliegen.

**Claims**

1. Chemically modified antisense oligonucleotides with sequences against the TAR sequence and leader sequence nt 21-53, 74-161, 202-279 and the second and third exon of the Tat gene nt 5368-5403, 5421-5548, 5583-5617 and nt 7967-8366, 8385-9183 of the HIV I genome.

2. Antisense oligonucleotides according to Claim 1 against the TAR sequence nt 21-53 and Tat sequence 5368-5403, 5421-5548, 5583-5617.

3. Antisense oligonucleotides according to Claims 1 and 2 from the group:

```
a) TAA CGC CTA TTC TGC TAT GTC GAC        (5366-5389)
b) TCT AGT CTA GGA TCT ACT GG             (5417-5436)
c) GGC TGA CTT CCT GGA TGC TT             (5444-5463)
d) GCA ATG AAA GCA ACA CTT TT             (5493-5512)
e) GAG TCT GAC TGC CTT GAG GA             (5584-5603)
f) CTT TGA TAG AGA AAC TTG AT             (5604-5623)
g) GCT CCC AGG CTC AGA TC                    (21-37)
h) CCA GAG AGC TCC CAG GC                     (28-44)
```

4. Antisense oligonucleotides according to Claims 1 to 3, in which one, several or all internucleotide phosphodiester linkages are in the form of phosphorothioates or methylphosphonates or both chemical modifications.

5. Medicament containing the antisense oligonucleotides according to Claims 1 to 4.

6. Antisense oligonucleotides according to Claims 1 to 4 for use for the therapeutic treatment of HIV infections.

7. Use of the antisense oligonucleotides according to Claims 1 to 4 including the complementary sequences as gene probe.

8. Use of antisense oligonucleotides with sequences according to Claim 3 including their complementary sequences as gene probes, where they are in the form of diesters.

**Revendications**

1. Oligonucléotides anti-sens modifiés chimiquement, comportant des séquences contre la séquence de TAR et la séquence leader nt 21-53, 74-161, 202-279 et le deuxième et le troisième exon du gène de Tat nt 5368-5403, 5421-5548, 5583-5617 et nt 7967-8366, 8385-9183 du génome de VIH-I.

2. Oligonucléotides anti-sens suivant la revendication 1 contre la séquence TAR nt 21-53 et la séquence Tat 5368-5403, 5421-5548, 5583-5617.

**3.** Oligonucléotides anti-sens suivant les revendications 1 et 2, du groupe :

| | |
|---|---|
| a) TAA CGC CTA TTC TGC TAT GTC GAC | (5366-5389) |
| b) TCT AGT CTA GGA TCT ACT GG | (5417-5436) |
| c) GGC TGA CTT CCT GGA TGC TT | (5444-5463) |
| d) GCA ATG AAA GCA ACA CTT TT | (5493-5512) |
| e) GAG TCT GAC TGC CTT GAG GA | (5584-5603) |
| f) CTT TGA TAG AGA AAC TTG AT | (5604-5623) |
| g) GCT CCC AGG CTC AGA TC | ( 21-37) |
| h) CCA GAG AGC TCC CAG GC | ( 28-44) |

**4.** Oligonucléotides anti-sens suivant les revendications 1 à 3, dans lesquels une, plusieurs ou la totalité des liaisons diester phosphoré internucléotidiques sont présentes sous forme de phosphorothioates ou de méthylphosphonates ou des deux formes chimiques.

**5.** Médicament contenant les oligonucléotides anti-sens suivant les revendications 1 à 4.

**6.** Oligonucléotides anti-sens suivant les revendications 1 à 4, destinés à être utilisés pour le traitement thérapeutique d'infections à VIH.

**7.** Utilisation des oligonucléotides anti-sens suivant les revendications 1 à 4, y compris les séquences complémentaires, comme sondes géniques.

**8.** Utilisation d'oligonucléotides anti-sens à séquences suivant la revendication 3, y compris leurs séquences complémentaires, comme sondes géniques, lesquelles se présentent sous forme de diester.

FIG.1

5´-Ende ————————————————tat-mRNA-Sequenz———————————3´-Ende

FIG. 2

FIG. 2a

FIG.2b

FIG. 3a

FIG. 3b